# EUROPEAN PATENT APPLICATION

(11) **EP 1 656 911 A1**
(43) Date of publication of application: **17.05.2006**
(21) Application number: 04425836.6
(22) Date of filing: 12.11.2004
(51) Int. Cl.: A61F 2/80, A61F 2/78

(54) **Device with a variable-volume ischial-containment socket**

(71) Applicant: Rizzoli Ortopedia S.p.A., 40054 Budrio (IT)
(72) Inventor: Varroni, Gianpaolo, 40134 Bologna (IT); Volta, Stefano, 40069 Zola Predosa (IT)
(74) Representative: Jorio, Paolo

(57) **Abstract**

Described herein is a device (10) for providing a variable-volume ischial-containment socket for patients who have recently undergone transfemoral amputation. The device (10) is characterized in that it envisages a substantially cup-shaped main body (11) designed to house within it a substantially tile-shaped element (14). The device (10) moreover comprises an adjustment device (16), which, by acting on the element (14), varies the extent of an internal space (13) designed to house a stump.

## Description

The present invention relates to a device for providing a variable-volume ischial-containment socket for patients who have recently undergone transfemoral amputation.

As is known, following upon amputation of a lower limb the patient is left with an oedematous stump.

Consequently, prior to application of the definitive prosthesis it is necessary either to await volumetric reduction of the stump, for example seeking to stabilize it by means of physiotherapy sessions, or else, alternatively, an adjustable socket is applied at an early stage, which has the feature of being able to follow the reduction in volume of the stump up to its stabilization and to shape the stump itself, as required by the definitive socket.

So far there exist two types of socket devices for patients who have undergone transfemoral amputation:
(Case 1): devices that use a quadrilateral socket, which is oval-shaped in the mediolateral direction; in this case, a counterthrust directed in the ventral and proximal part of the socket (Scarpa's triangle) has the function of stabilizing the ischial tuberosity in its position of rest; or else
(Case 2): devices that envisage an ischial-containment socket, characterized in that the ischium and the ischial branch are contained and immobilized within it; moreover, in this kind of socket there is an accentuated total contact that becomes also a terminal point of rest in the case where the apex of the stump so allows; moreover, this type of socket is characterized by a shape elongated in an anteroposterior direction and presents an anterior stabilizing counterthrust in the "Scarpa's triangle" that is less accentuated than in the quadrilateral socket (compare Case 1).

Since the action produced by the shape of the ischial-containment socket (Case 2) enables:
- a greater stability of the stump within the socket;
- a reduction of the loads concentrated on the stump;
- a better alignment of the socket with respect to the line of loading;
- a better control of the prosthesis by the patient both in the static position and in the dynamic position; and
- a reduction of the anterior thrust in the area of the "Scarpa's triangle".

A temporary ischial-containment socket has been devised for patients who have undergone transfemoral amputation, which will have the same characteristics as a definitive ischial-containment socket, but which, at the same time, will have the characteristics of following the reduction in the volume of an oedematous stump resulting from a recent amputation so as to render the stump conformable for a definitive ischial-containment socket.

Consequently, according to the present invention there is made available a device for providing a variable-volume ischial-containment socket for patients who have recently undergone transfemoral amputation, as is defined in the characteristics specified in Claim 1.

The present invention will now be described with reference to the annexed drawings, which illustrate a non-limiting example of embodiment thereof and in which:
- Figure 1 illustrates as a whole an ischial-containment socket device forming the subject of the present invention;
- Figure 2 represents an exploded view of the device of Figure 1; and

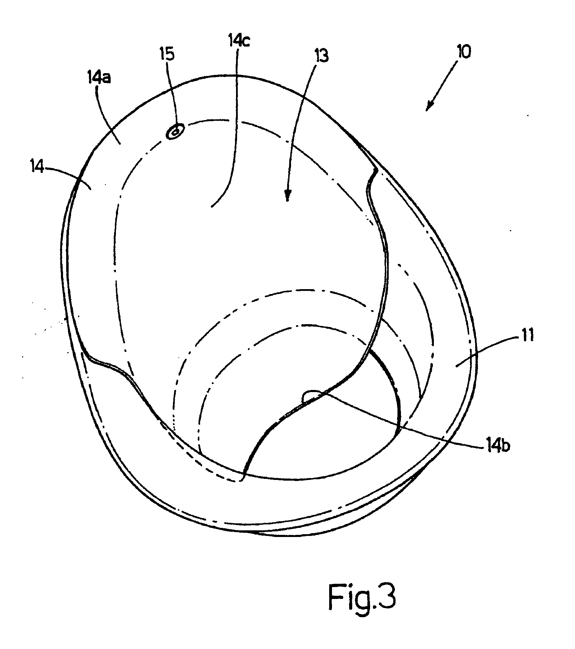

- Figure 3 shows a view from above of the device represented in Figures 1 and 2.

In the embodiment of the invention designated in the attached figures as a whole by 10 is a device for providing a variable-volume ischial-containment socket for patients who have recently undergone transfemoral amputation.

The device 10 comprises a main socket body 11, which is substantially cup-shaped according to the widely known teachings of correct ischial containment. The main body 11 is advantageously made of a single piece and of a plastic material, for example homopolymer polypropylene.

Furthermore, the main body 11 has an internal wall 11a and is fixed, adopting usual methods, to a rest 12 (illustrated only partially for convenience of representation).

The desired action of stump containment (not illustrated) and of definition of an internal space 13 for housing the stump itself has been obtained in an inventive way by installing a tile-shaped element 14 within the main body 11.

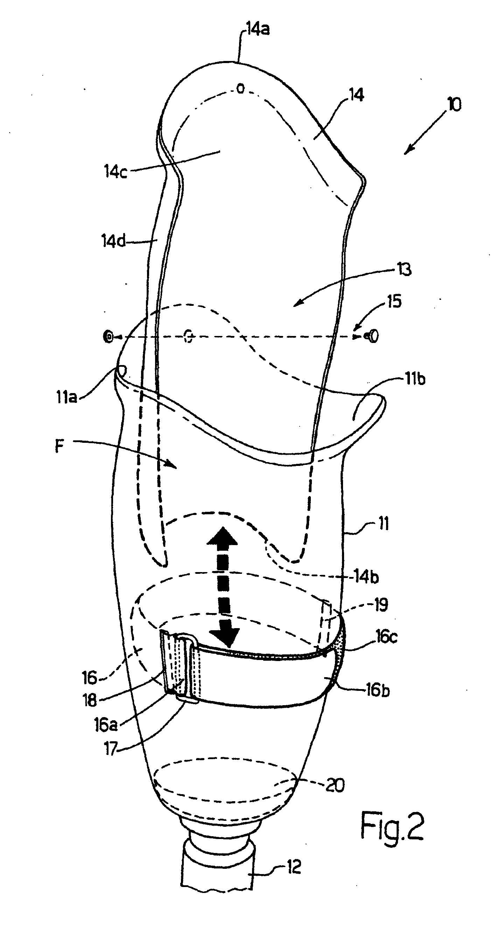

The element 14 has a top end 14a and a bottom end 14b. The top end 14a is hinged to the wall 11a of the main body 11 by means of a pin 15, illustrated in particular in the exploded view of Figure 2.

More in particular, the element 14 is pivoted on the supratrochanteric flange of the load-bearing structure of the cup-shaped main body 11.

Furthermore, the element 14 is advantageously made of a single piece and of plastic material, for example polyethylene LLDPE. Entering into greater detail, we can say that the internal space 13 is defined by a front portion 11b of the aforementioned internal wall 11a and by an internal wall 14c of the element 14.

To obtain a perfect containment of the stump it will be necessary to reduce the internal space 13 proportionally, continuing to maintain contact of the stump with the top profile of the main body 11 and continuing to tighten the stump itself between the wall 14c of the element 14 and the portion 11b of the wall 11a.

In an inventive way, to obtain the required variations of the internal space 13 the system illustrated in the attached figures is applied.

For this purpose, one or more straps 16 are provided according to the length of the stump.

The strap 16 comprises a first end 16a fixed to a buckle 17 and a second end 16b having reversible fixing means, such as for example ones made of Velcro®.

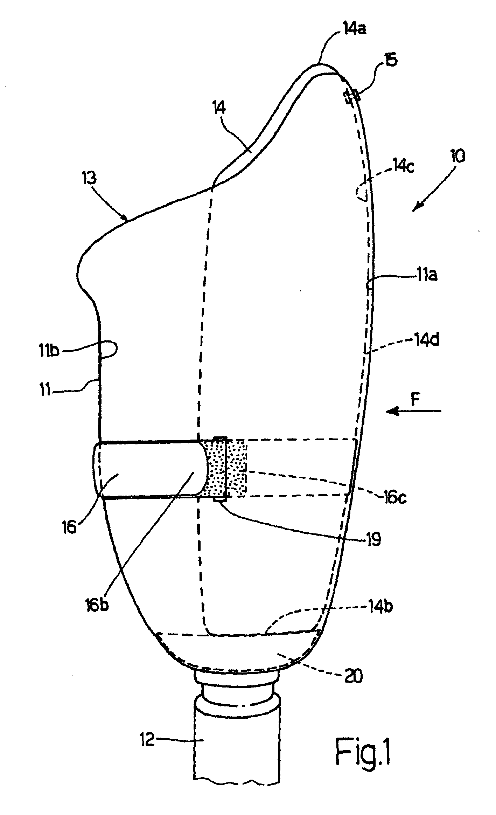

As illustrated in Figures 1 and 2, the strap 16, in use, is first inserted into a substantially vertical slit 18 and then inserted into the space between the internal wall 11a of the main body 11 and an external wall 14d of the element 14.

The strap 16 is then made to pass in a slit 19 substantially parallel to the slit 18, and once it comes out again on the outside of the main body 11 is rewound about the buckle 17.

In use, once the dimensions of the internal space 13 have been established, the end 16b of the strap 16 is fixed to a portion 16c of the strap 16 itself, which is also coated with Velcro®.

It is evident how the user, by tightening or releasing the strap 16 will bend the element 14 about the fulcrum 15, thus managing to reduce, or if necessary enlarge, the internal space 13 adapting to the variations in volume of the stump.

More in particular, by releasing the end 16b from the portion 16c and pulling the strap 16, there is obtained an action of the latter on the wall 14d of the element 14 in the direction indicated by an arrow F (Figure 2). As a result of this action, the lower portion of the element 14 is induced to bend about the pin 15, thus reducing the internal space 13 designed to house the stump.

The end 14b of the element 14 brushes against a cushioned element 20 acting as a buffer, which is housed on the bottom of the main body 11, on which the end of the stump rests in use.

In a second embodiment (not illustrated), the reduction of the internal space 13 (starting from a maximum internal space 13 that exists in the case where the wall 14d rests almost completely on the wall 11a) is obtained by inserting appropriate thicknesses of non-compressible material between the wall 11a and the wall 14c.

Finally, in a third embodiment (not illustrated) the reduction of the internal space 13 (starting from a maximum internal space 13 that exists in the case where the wall 14d rests almost completely on the wall 11a) is obtained by inserting between the wall 11a and the wall 14c a container designed to contain air, other gases, gel, silicone, or liquids.

The main advantage of the present device consists in the creation of an ischial-containment socket device that is easy to produce (only three pieces, namely in the example illustrated and described herein, a main body, a tile-shaped element, and a strap) and one which enables a convenient and effective adjustment of the internal space for containing the stump.

## Claims

1. A device (10) for providing a variable-volume ischial-containment socket for patients who have recently undergone transfemoral amputation; said device (10) being **characterized in that** it envisages a substantially cup-shaped main body (11) designed to house within it a basically tile-shaped element (14), and **in that** it comprises means (16), which, by acting on said element (14), adjust the extent of an internal space (13) designed to house a stump.

2. The device (10) according to Claim 1, **characterized in that** the element (14) is hinged to said main body (11) by means of a pin (15).

3. The device (10) according to any one of the preceding claims, **characterized in that** said main body (11) is made of a plastic material in a single piece.

4. The device (10) according to Claim 2, **characterized in that** said means (16) are designed to bend said element (14) about said pin (15).

5. The device (10) according to Claim 1, **characterized in that** said means (16) comprise at least one strap (16) provided with reversible fixing means (16b, 16c).

6. The device (10) according to Claim 1, **characterized in that** said means (16) comprise thicknesses of non-compressible material.

7. The device (10) according to Claim 1, **characterized in that** said means (16) comprise at least one container designed to contain a gas or a liquid.

8. The device (10) according to Claim 1, **characterized in that** said means (16) comprise at least one container designed to contain a substance in the form of a gel.
